# EUROPÄISCHE PATENTSCHRIFT

(11) **EP 0 663 183 B1**
(45) Veröffentlichungstag und Bekanntmachung des Hinweises auf die Patenterteilung: **20.10.1999**
(21) Anmeldenummer: 95100412.6
(22) Anmeldetag: 13.01.1995
(51) Int. Cl.: A61B 10/00

(54) **Biopsienadel und Stilett hierfür**
Biopsy needle and stylet
Aiguille de biopsie avec un stylet

(30) Priorität: 14.01.1994 US 180972
(43) Veröffentlichungstag der Anmeldung: 19.07.1995
(73) Patentinhaber: HAAGA, John, R., Chagrin Falls, OH 44022 (US)
(72) Erfinder: HAAGA, John, R., Chagrin Falls, OH 44022 (US)
(74) Vertreter: Hennicke, Albrecht, Dipl.-Ing.

(56) Entgegenhaltungen:
- EP-A- 0 153 047
- WO-A-91/01112
- DE-C- 935 625
- GB-A- 1 393 068
- US-A- 2 496 111
- US-A- 3 995 619
- US-A- 5 127 419

## Beschreibung

Die Erfindung betrifft ein chirurgisches Instrument, nämlich eine Biopsienadel und ein Stilett hierfür, womit das Ausschneiden und Entnehmen von Gewebeproben bei einem Biopsieverfahren erleichtert und verbessert werden kann.

Eine Biopsienadel derjenigen Art, auf die sich die vorliegende Erfindung bezieht, ist eine Seitenschneidnadel, wie sie beispielsweise in dem US-Patent Nr. 3 477 423 von Griffith dargestellt und beschrieben ist. Bei einer solchen Seitenschneidnadel ist ein massives Stilett teleskopartig verschiebbar in einer rohrförmigen, inneren Schneidkanüle angeordnet, die ihrerseits teleskopartig in einer rohrförmigen Außenkanüle verschiebbar ist, in der das Stilett und die Innenkanüle relativ zueinander und relativ zu der Außenkanüle axial verschoben und gedreht werden können. Das Stilett ist in seinem distalen Teil mit einer eine Probe ausschneidenden Aufnahme oder mit einer Gewebekammer versehen, mit der eine an der Biopsiestelle ausgeschnittene Gewebeprobe entnommen werden kann. Hierbei wird die Seitenschneidnadel in einen Patienten eingeführt, bis das distale Ende der Außenkanüle die kranke Stelle erreicht, wo die Biopsieprobe entnommen werden soll. Dann wird das Stilett aus der äußeren und inneren Kanüle in das kranke Organ zu der Biopsiestelle vorgeschoben. Das Gewebe an dieser Stelle dringt in die Probenaufnahmekammer ein und dann wird die innere Kanüle gegenüber der äußeren Kanüle ausgeschoben und über das Stilett geschoben, um das Gewebe in der Probenkammer abzuschneiden und die Aufnahmekammer zu verschließen und hierdurch die Probe hierin einzufangen, so daß sie aus der Biopsiestelle entnommen werden kann. Die Entnahme geschieht durch Zurückziehen des Stiletts und der inneren Schneidkanüle in die äußere Kanüle und dann durch Herausziehen der Biopsienadel aus dem Patienten.

Eine solche Biopsienadel nach dem Oberbegriff des Anspruches 1 ist auch in der EP 153 047 A offenbart. Bei dieser Biopsienadel ist das Stilett in einer inneren Schneidkanüle geführt, die beim Entnehmen der Gewebeprobe dazu eingesetzt wird, den aus der Aufnahmekammer des Stilettes überstehenden Teil an Gewebe abzuschneiden. Die Schneidkanüle ist dabei innerhalb eines Führungsrohres angeordnet. Die Aufnahmekammer ist im Querschnitt in Längsrichtung im wesentlichen rechteckig ausgebildet, d.h. die beiden Stirnwände verlaufen fast senkrecht nach unten zu dem Boden der Aufnahmekammer.

Bisher wurde die schneidende Aufnahmekammer im Stilett einer Biopsienadel von einer sich parallel zur Achse des Stiletts in Axialrichtung erstreckenden Bodenwand und von Stirnwänden gebildet, die im axialen Abstand voneinander senkrecht zur Bodenwand und zur Stilettachse angeordnet waren. Wenn das Stilett in denjenigen Bereich vorgeschoben wird, aus dem die Gewebeprobe entnommen werden soll, dringt das Gewebe in die Aufnahmekammer ein, während das Stilett durch das Gewebe vordringt. Das Gewebe wird hierbei durch Vorschieben der inneren Schneidkanüle über das Stilett abgeschnitten und in der Aufnahmekammer eingeschlossen.

Bei einer derart konstruierten Aufnahmekammer kam es nicht selten vor, daß die gezogene Gewebeprobe nur die Hälfte oder Dreiviertel der Aufnahmekammer ausfüllte. Dieser Fehler ist darauf zurückuzuführen, daß es schwierig ist, das Stilett an der Biopsiestelle ruhig zu halten, während die Schneidkanüle über das Stilett vorgeschoben wird. Wenn die einzelnen Teile der Biopsienadel von Hand betätigt werden, kann es nämlich geschehen, daß beim Vorschieben der Schneidkanüle gleichzeitig auch das Stilett an der Biopsiestelle vorgeschoben oder zurückgezogen wird. Hierdurch wird ein Teil der von der schneidenden Aufnahmekammer zunächst aufgenommenen Probe wieder aus der Kammer herausgedrückt, während die Schneidkanüle die Gewebeprobe beim Vorschieben abschneidet.

Biopsienadeln der vorstehend beschriebenen Art werden häufig auch mit einer Pistole betätigt, wobei die Verschiebung der einzelnen Teile der Biopsienadel während der Biopsie automatisch vor sich geht. Bei solchen selbsttätig arbeitenden Pistolen gibt es einen Rückstoß, der das Stilett an der Biopsiestelle beim Vorschieben der Schneidkanüle vorwärts drücken will. Auch hierbei wird ein Teil des in der Aufnahmekammer bereits vorhandenen Gewebes aus dieser wieder herausgedrückt.

Es ist anzunehmen, daß dieser Nachteil mit der Konstruktion und Wirkungweise des oben beschriebenen Stiletts zusammenhängt und auf die senkrechte Anordnung der Stirnwände zu der Bodenwand der Probenaufnahmekammer zurückgeht. Wenn nämlich das Stilett relativ zu der Gewebestelle beim Vorschieben der Schneidkanüle vorgeschoben oder zurückgezogen wird, bewegen sich die Stirnwände an den einander gegenüberliegenden Enden der Aufnahmekammer gegen die Gewebeprobe und die senkrechten Wände lenken hierbei das Gewebe aus der Aufnahmekammer seitlich nach außen. Hierdurch wird die Menge des Gewebes, das beim Vorschieben der Schneidkanüle in der Aufnahmekammer eingeschlossen wird, im Vergleich zum optimalen Fassungsvermögen der Aufnahmekammer reduziert.

Aus dem US-Patent Nr. 5 127 419 ist eine Biopsienadel bekannt, bei der ein Stilett in Längsrichtung in einer Kanüle verschiebbar geführt ist. Das Stilett und die Kanüle sind jeweils über Betätigungszapfen in Längsrichtung bewegbar, so daß nach dem Einführen des Stilettes die Kanüle eine Gewebeprobe abschneiden kann, die in eine Aufnahmekammer in dem Stilett eingetreten ist.

Die Aufnahmekammer ist in ihrem Bodenbereich in Längsrichtung etwas länger als in dem seitlichen Öffnungsbereich. Zwar sind das Stilett und die Kanüle in Längsrichtung verschiebbar, sie können jedoch nicht gegeneinander verdreht werden, da eine Drehbewegung durch in Schlitzen geführte Betätigungszapfen verhindert wird.

Die GB-A-1 393 068 offenbart Biopsienadeln mit Stiletten in verschiedenen Ausführungsformen. Die Stilette weisen in ihrem distalen Endbereich jeweils eine Aufnahmekammer für Gewebeproben auf. Die Aufnahmekammer kann an ihrem distalen Ende halbkreisförmig ausgebildet sein, so daß dieser Endbereich im Stilett eine Tasche bildet.

Ferner ist aus dem US-Patent Nr. 2 496 111 eine Biopsienadel bekannt, die ein Stilett mit Widerhaken, eine innere hohle Schneidnadel und ein äußeres Führungsrohr aufweist. Zum Entnehmen einer Gewebeprobe wird das Stilett in den Körper eingeführt und durch die nachfolgende Bewegung der Schneidnadel wird die Gewebeprobe zwischen der Schneidnadel und dem Stilett eingeschlossen und kann so entnommen werden.

Aufgabe der Erfindung ist es, eine Biopsienadel mit Stilett so auszubilden, daß eine die Aufnahmekammer der Nadel bzw. des Stiletts vollständig ausfüllende Probe aus dem zu untersuchenden Gewebe ausgeschnitten und in der Aufnahmekammer während des Biopsieverfahrens und bei Entnahme der Gewebeprobe sicher festgehalten werden kann.

Diese Aufgabe wird gemäß der Erfindung durch eine Biopsienadel mit den Merkmalen des Anspruches 1 gelöst. Erfindungsgemäß weist die Aufnahmeöffnung des Stiletts eine parallel zu dessen Achse verlaufende Bodenwand und distale und proximale Stirnwände auf, die sich zwischen der Bodenwand und der äußeren Oberfläche des Stiletts erstrecken und eine radial im Abstand von der Bodenwand befindliche Eintrittsöffnung und einen inneren Bereich begrenzen, der in Axialrichtung größer ist als die Eintrittsöffnung. Eine solche Ausbildung der Aufnahmekammer wird dadurch erreicht, daß sowohl die distale als auch die proximale Stirnwand der Kammer, von der Eintrittsöffnung der Aufnahmekammer in distaler bzw. proximaler Richtung zurückspringen und die Aufnahmekammer hierdurch einen von der Eintrittsöffnung aus radial weiter innenliegenden Bereich aufweist, dessen Abmessung in Axialrichtung größer ist als die axiale Länge der Eintrittsöffnung. Die Probe kann sich deshalb in der Kammer in Längsrichtung ausdehnen und wird dort beim Abschneiden sicher festgehalten.

Die zurückspringende distale Stirnwand der Aufnahmekammer bildet am distalen Ende eine Kerbe oder Nute, die ein Zurückgleiten des Stiletts im Gewebe verhindert, während die Schneidkanüle vorgeschoben wird. Da außerdem die radiale Außenkante der Stirnwand eine im wesentlichen elliptische Kontur hat und sich nicht in einer zur Stilettachse senkrechten Ebene befindet, wird das Ausschneiden der Gewebeprobe beim Drehen des Stiletts erleichtert. Die zurückspringende proximale Stirnwand der Aufnahmekammer bildet ebenfalls eine Nute, die verhindert, daß das Stilett beim Vorschieben der Schneidkanüle weiter in die Biopsiestelle vorgeschoben wird. Durch die in Axialrichtung vorspringenden Außenkanten der Stirnwände wird das Ausschneiden der Gewebeprobe weiter erleichtert.

Die Nuten oder Kerben, die durch die zurückspringende Ausbildung der distalen und proximalen Stirnwände der Aufnahmekammer erzeugt werden, haben den weiteren Vorteil, daß die Gewebeprobe in der schneidenden Aufnahmekammer sicher festgehalten und gegen seitliches Herausgleiten gesichert wird. Hierdurch wird das Ausschneiden des Gewebes erleichtert, wenn die Schneidkanüle vorgeschoben wird. Gleichzeitig wird eine größtmögliche Gewebemenge in der Aufnahmekammer eingeschlossen und kann aus der Biopsiestelle entnommen werden. Hierbei wird die Kerbe oder Nute am proximalen Ende der Aufnahmekammer in das Gewebe gedrückt und die zurückspringende proximale Stirnwand drückt das Gewebe radial ins Innere der Aufnahmekammer und in die Ecke zwischen der proximalen Stirnwand und der Bodenwand der Aufnahmekammer, so daß das Gewebe daran gehindert wird, wieder aus der Aufnahmekammer herauszugleiten. Die Nute oder Kerbe am distalen Ende der Aufnahmekammer erleichtert das Füllen der Aufnahmekammer in Axialrichtung mit dem Gewebe während des Biopsieverfahrens dadurch, daß die zurückspringende distale Stirnwand der Aufnahmekammer auch dazu dient, Gewebe in der Kerbe einzufangen, wobei das Gewebe an einer Verschiebung radial aus der Aufnahmekammer beim Vorschieben der Schneidkanüle gehindert wird.

Die erfindungsgemäße Ausführung hat den Vorteil, daß die schneidende Aufnahmekammer das Stilett an einer Biopsiestelle im Gewebe festhält und seine Axialverschiebung gegenüber dem Gewebe während einer Biopsie verhindert. Ein weiterer Vorteil besteht darin, daß die Gewebemenge optimiert wird, die beim Biopsieverfahren aus der Biopsiestelle entnommen werden kann.

Durch die erfindungsgemäße Ausbildung der schneidenden Aufnahmekammer ergibt sich der weitere Vorteil, daß die Gewebeprobe in der Aufnahmekammer beim Vorschieben einer Schneidkanüle zum Abschneiden und Einschließen der Probe in der Kammer festgehalten wird.

Weitere Merkmale und Vorteile der Erfindung ergeben sich aus der nachfolgenden Beschreibung und den Zeichnungen, in denen eine bekannte Ausführungsform und Ausführungsformen nach der Erfindung an Beispielen näher erläutert sind. Es zeigt:
- Fig. 1: ein Biopsienadelstilett bekannter Art in einer Seitenansicht und teilweise im Längsschnitt,
- Fig. 2: den Gegenstand der Fig. 1 in einem Querschnitt nach Linie 2-2,
- Fig. 3: ein Biopsienadelstilett nach der Erfindung in einer Seitenansicht und teilweise im Längsschnitt,
- Fig. 4: den Gegenstand der Fig. 3 in einem Querschnitt nach Linie 4-4,
- Fig. 5: eine Biopsienadel nach der Erfindung mit Außenkanüle, Schneidkanüle und Stilett in zusammengebautem Zustand vor dem Gebrauch der Nadel in einem Längsschnitt und teilweise in einer Seitenansicht,
- Fig. 6 u. 7: die Biopsienadel nach Fig. 5 beim Entnehmen der Biopsieprobe, bei dem die einzelnen Teile der Nadel verschiedene Stellungen einnehmen und
- Fig. 8: eine andere Ausführungsform eines Biopsienadelstiletts nach der Erfindung in einer Seitenansicht und teilweise im Längsschnitt.

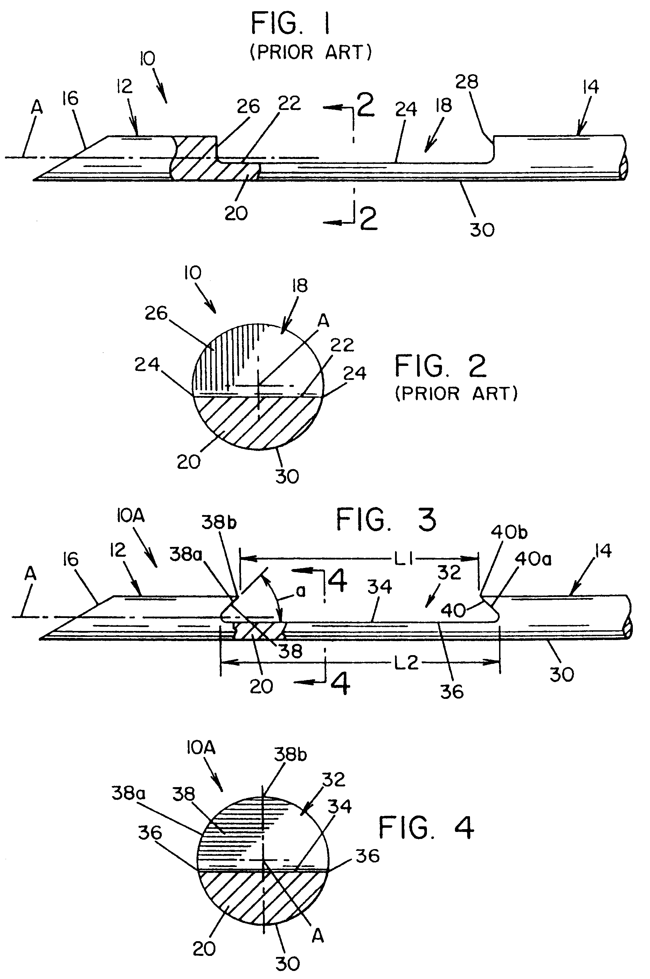

In den Fig. 1 und 2 ist ein Biopsienadelstilett 10 bekannter Art dargestellt, das einen massiven Kreisquerschnitt mit einer Achse A und einem Durchmesser hat, mit dem das Stilett, wie an sich bekannt, in einer rohrförmigen Innen- oder Schneidkanüle einer Biopsienadel gleiten kann und drehbar ist. Das Stilett 10 hat einen distalen Teil 12 und einen proximalen Teil 14, die sich axial zur Mitte hin erstrecken und von denen das axial äußere Ende des distalen Teiles 12 abgeschrägt ist und eine distale Spitze oder ein distales Ende 16 bildet. Das Stilett ist radial und axial an einer Stelle zwischen dem distalen Ende 16 und dem proximalen Teil 14 ausgeschnitten und bildet hier eine Probenaufnahme 18 und einen massiven Teil 20, der die Aufnahmekammer überspannt. Die Aufnahmekammer 18 hat eine parallel zur Achse A verlaufende Bodenwand 22, die in Axialrichtung sich erstreckende, entgegengesetzt gerichtete Seitenkanten 24 aufweist, welche Schneidkanten bilden, mit denen eine Probe an der Biopsiestelle ausgeschnitten werden kann, wenn das Stilett gedreht wird. Die Aufnahmekammer 18 hat ferner eine distale Stirnwand 26 und eine proximale Stirnwand 28, die beide senkrecht zur Bodenwand 22 stehen und sich zwischen der Bodenwand und der Außenfläche 30 des Stiletts erstrecken.

Die Fig. 3 und 4 zeigen eine Abwandlung der Aufnahmekammer in einem Stilett der vorhergehenden Art, um ein Stilett 10A nach der Erfindung zu schaffen. Mit Ausnahme der Aufnahmekammer entspricht das Stilett 10A dem oben beschriebenen Stilett 10, wobei in den Fig. 3 und 4 gleiche Bezugszeichen verwendet sind, um entsprechende Teile des Stiletts zu bezeichnen. Bezugnehmend auf die zuletzt genannten Fig. ist das Stilett 10A mit einer Aufnahmekammer 32 versehen, die eine sich in Axialrichtung erstreckende Bodenwand 34 aufweist, die parallel zur Achse A verläuft und sich in Axialrichtung erstreckende, einander gegenüberliegende Seitenkanten 36 aufweist, die den Seitenkanten 24 des Stiletts 10 in den Fig. 1 und 2 entsprechen. Die Aufnahmekammer 32 hat eine distale Stirnwand 38 und eine proximale Stirnwand 40, von denen sich jede zwischen der Bodenwand 34 und der Außenfläche 30 des Stiletts erstreckt und von denen jede gegenüber dem entsprechenden distalen Teil 12, bzw. dem proximalen Teil 14 des Stiletts nach innen zurückspringt. Die nach innen zurückspringenden Stirnwände 38 und 40 bilden eine Aufnahmekammer 32, die im radialen Abstand von der Bodenwand 34 eine Eintrittsöffnung mit einer axialen Länge L1 hat und einen von der Eintrittsöffnung radial weiter nach innen liegenden Bereich aufweist, der eine größere axiale Länge L2 hat, die größer ist als die Länge L1.

Bei der in den Fig. 3 und 4 dargestellten Ausführungsform sind die Stirnwände 38 und 40 eben und ihre einspringende Ausbildung ist dadurch erreicht, daß jede dieser Wände unter einem spitzen Winkel a gegenüber der Bodenwand 34 geneigt ist. Der Winkel a liegt zwischen 15° und 85° und beträgt vorzugsweise etwa 45°. Die Stirnwand 38 hat eine radiale Außenkante 38a, die eine elliptische Kontur aufweist, wenn man sie senkrecht zur Bodenwand 34 sieht, und die Wand 40 hat eine radiale Außenkante 40a der gleichen Kontur. Die Außenkanten 38a und 40a verschneiden sich mit einer zur Bodenwand 34 senkrechten Axialebene an den Punkten 38b bzw. 40b und bestimmen die Länge L1 der Eintrittsöffnung der Aufnahmekammer.

Obgleich die Verschneidungen zwischen den Wänden 38 und 40 und den entsprechenden Enden der Bodenwand 34 ausgerundet dargestellt sind, ist doch zu erkennen, daß diese Verschneidungen auch durch scharfe Ecken oder durch Ausrundungen mit größerem Radius als dargestellt ausgebildet sein können.

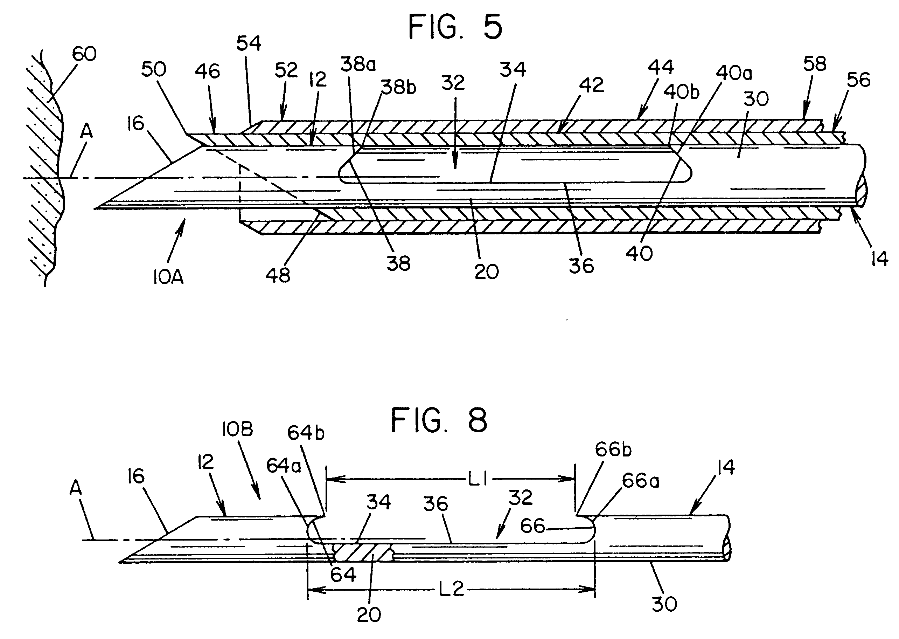

Wendet man sich jetzt der Fig. 5 zu, so erkennt man, daß das Stilett 10A mit einer rohrförmigen, inneren Schneidkanüle 42 und einer rohrförmigen äußeren Kanüle 44 zusammengebaut ist, die eine Biopsienadel bilden, in der das Stilett und die Kanülen zueinander koaxial angeordnet und so relativ zueinander gelagert sind, daß sie axial gegeneinander verschoben und in Umfangsrichtung gegeneinander verdreht werden können. Die innere Schneidkanüle 42 hat einen distalen Teil 46 mit einer abgeschrägten, distalen Schneidkante 48, die an ihrem distalen Ende eine Schneidspitze 50 aufweist. Die äußere Kanüle 44 hat einen distalen Teil 52 mit einem abgeschrägten distalen Ende 54. Die innere Kanüle 42 hat einen proximalen Teil 56 und die äußere Kanüle 44 hat einen proximalen Teil 58. Obgleich dies nicht dargestellt ist, erkennt man doch, daß die proximalen Enden der Kanülen und des Stiletts mit Handhaben od.dgl. versehen sind, um die Handbetätigung der einzelnen Nadelteile relativ zueinander zu erleichtern. Alternativ haben die proximalen Enden auch die erforderlichen Anschlüsse zum Verbinden der einzelnen Nadelteile mit einer Pistole, mit der die Betätigung der Nadel automatisiert wird.

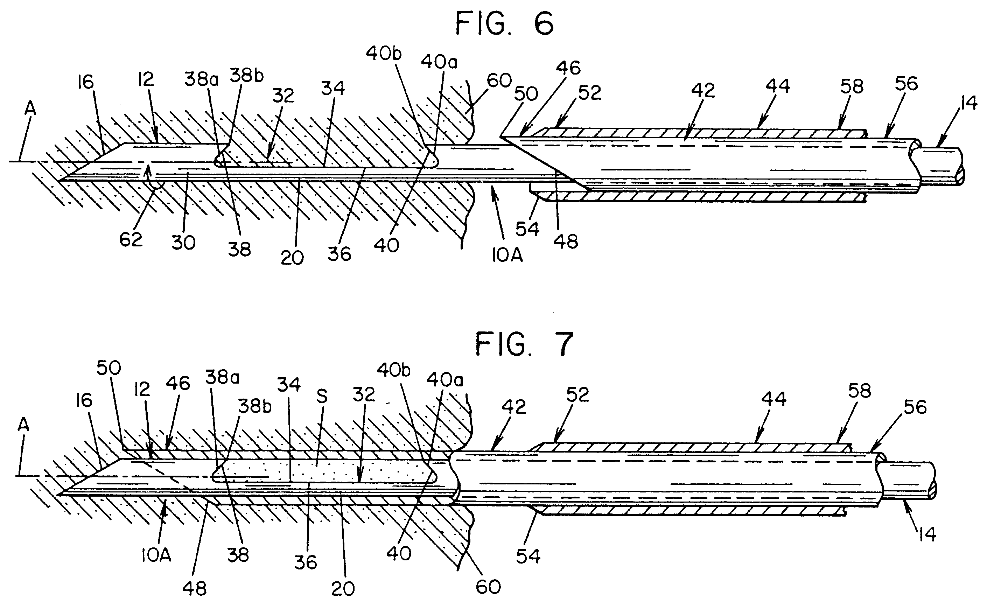

Im zusammengebauten Zustand sind die Schneidkanüle 42 und das Stilett 10A zwischen einer eingeschobenen und einer ausgezogenen Stellung gegenüber der äußeren Kanüle 44 verschiebbar und sie befinden sich vor Gebrauch in ihrer zurückgezogenen Stellung, die in Fig. 5 dargestellt ist. Bei Gebrauch wird die Nadel bei zurückgezogener innerer Kanüle und zurückgezogenem Stilett in den Körper eines Patienten eingeführt, bis die distalen Enden der einzelnen Teile der Nadel sich neben einer kranken Stelle 60 befinden, aus der eine Biopsieprobe entnommen werden soll, wie dies in Fig. 5 dargestellt ist. In dieser Stellung angekommen, wird das Stilett 10A gegenüber der äußeren und inneren Kanüle aus seiner zurückgezogenen Stellung axial in seine ausgezogene Stellung herausgeschoben, in der sich die Probenaufnahmekammer 32 an der Biopsiestelle im krankhaften Bereich 60 befindet, wie dies in Fig. 6 dargestellt ist. Wie dieser Fig. zu entnehmen ist, stabilisieren die gegenüber der Bodenwand 34 der Aufnahmekammer 32 zurückgebogenen Stirnwände 38 und 40 das Stilett 10A gegen Axialverschiebung in der kranken Stelle, da bei jeder Axialverschiebung die Stirnwände gegen die benachbarten Enden des Gewebes stoßen und dieses in die Aufnahmekammer nach innen gegen die Bodenwand 34 drücken. Dies hindert in vorteilhafter Weise die Endbereiche des Gewebes daran, beim vollständigen Abschneiden der Probe durch die Eintrittsöffnung hinausgedrückt zu werden. Ein solches Hinausdrücken würde einen Verlust eines Teiles der Gewebeprobe zur Folge haben, der sonst mit eingeschlossen und aus der Biopsiestelle entnommen werden würde.

Wenn sich das Stilett 10A in der krankhaften Veränderung 60 an der Biopsiestelle befindet, wie dies in Fig. 6 dargestellt ist, kann das Stilett um seine Achse A gedreht werden, wie dies durch den Pfeil 62 für eine der Kanten 36 der Bodenwand 34 und die Kanten 38a und 40a der Stirnwände 38 und 40 angedeutet ist, um das Gewebe an der Biopsiestelle abzutrennen. Hierbei haben die zurückgebogenen Stirnwände 38 und 40 der Aufnahmekammer 32 den weiteren Vorteil, daß sie beim Drehen des Stiletts 10A ein Ausschneiden des Gewebes erleichtern, im Vergleich zu dem Schnitt, der mit einem Stilett durchgeführt werden kann, bei dem die Stirnwände der Aufnahmekammer zu deren Bodenwand senkrecht stehen. Insoweit drückt die zurückgebogene Ausführung die benachbarten Endteile des Gewebes gegen die Bodenwand der Aufnahmeöffnung, wie dies oben beschrieben worden ist, während die Kanten 38a und 40a der Stirnwände beim Abschneiden des Gewebes bei einer Drehung des Stiletts um die Achse A wirksam sind. In jedem Falle wird die Schneidkanüle 42 relativ zur Außenkanüle 44 und zum Stilett 10A aus ihrer in Fig. 6 dargestellten zurückgezogenen Stellung in ihre in Fig. 7 dargestellte, Stellung axial nach außen geschoben, wenn sich das Stilett 10A an der Biopsiestelle befindet. Während dieser Bewegung der Schneidkanüle 42 schneiden die Schneidkante 48 und die Spitze 50 an ihrem distalen Ende das Gewebe an der Biopsiestelle in die Aufnahmekammer 32 hinein und die Kanüle 42 schließt bei ihrer Bewegung axial über die Aufnahmekammer hinweg eine Probe S in der Aufnahmekammer 32 ein, wie dies in Fig. 7 dargestellt ist.

Wie bereits oben erwähnt, erreicht die Größe der durch die Bewegung der Schneidkanüle 42 in die in Fig. 7 dargestellte Stellung eingefangenen Probe durch den Stabilisierungseffekt der zurückspringenden Stirnwände 38 und 40 ein Optimum.

Wenn die Gewebeprobe S in der Aufnahmekammer 32, wie oben beschrieben, eingeschlossen ist, werden das Stilett 10A und die Schneidkanüle 42 gemeinsam aus der kranken Stelle in die Außenkanüle 44 zurückgezogen, wie dies in Fig. 5 dargestellt ist. Dann wird die Nadel aus dem Patienten herausgezogen.

Fig. 8 zeigt eine andere Ausführungsform eines Stiletts nach der vorliegenden Erfindung. Bei dieser Ausführungsform ist das Stilett 10B mit dem oben beschriebenen Stilett 10A identisch, mit Ausnahme der Kontur der Stirnwände der Probenaufnahmekammer. Hierbei werden in Fig. 8 gleiche Bezugszeichen benutzt, um diejenigen Teile des Stiletts 10B zu bezeichnen, die denjenigen des Stiletts 10A entsprechen. Bei dieser Ausführungsform hat die schneidende Aufnahmekammer 32 distale und proximale Stirnwände 64 und 66, die von der Außenfläche 30 des Stiletts gegenüber dem jeweils entsprechenden distalen Teil 12 oder proximalen Teil 14 des Stiletts zurückgebogen sind. Die Stirnwände 64 und 66 haben zwischen der Bodenwand 34 der Aufnahmekammer und der Außenfläche 30 des Stiletts eine gebogene Kontur und haben sich in Umfangsrichtung erstreckende, radial außenliegende Seitenkanten 64a und 66a. Die Seitenkanten 64a und 66a verschneiden sich mit einer auf der Bodenwand 34 senkrecht stehenden Axialebene an den Punkten 64b und 66b und bilden die Aufnahmekammer, die eine Eintrittsöffnung mit einer axialen Länge L1 hat. Die zurückgebogenen Stirnwände 64 und 66 führen dazu, daß die Aufnahmekammer einen von der Eintrittsöffnung aus radial innenliegenden Bereich aufweist, der eine größere axiale Länge L2 hat, die größer ist als die Länge L1. Wie man aus der vorhergehenden Beschreibung des Stiletts 10A erkennt, verleihen die zurückgebogenen Stirnwände 64 und 66 dem Stilett 10B die gleichen Eigenschaften wie die Stirnwände 38 und 40 des Stiletts 10A beim Gebrauch einer Biopsienadel, die ein Stilett 10B aufweist.

Obgleich im vorstehenden großer Wert auf die Konstruktionen der bevorzugten Ausführungsformen der Stiletts gelegt wurde, erkennt man doch, daß auch andere Konstruktionen und Abwandlungen der beschriebenen Ausführungsform möglich sind, ohne den Rahmen der Erfindung zu verlassen. Beispielsweise könnten die Stirnwände der Gewebeaufnahmekammer des Stiletts auch V-förmig sein, wobei der Scheitel des V parallel zur Bodenwand der Aufnahmekammer verläuft und zwischen der Bodenwand und der Außenfläche des Stiletts angeordnet sein könnte. Außerdem muß die zurückgebogene Ausbildung der distalen und proximalen Stirnwände der Aufnahmekammer nicht die gleiche sein und sie kann beispielsweise auch eine Kombination einer ebenen geneigten Wand, wie sie bei dem Stilett 10A dargestellt ist, mit einer gebogenen Wand sein, wie sie bei dem Stilett 10B gezeigt ist. Als weiteres Beispiel müssen die spitzen Winkel zwischen den Stirnwänden und der Bodenwand des Stiletts 10A nicht immer die gleichen sein. Diese und andere Abwandlungen der bevorzugten Ausführungsformen, ebenso wie andere Ausführungsbeispiele der Erfindung sind für Fachleute offensichtlich und naheliegend und fallen mit in den Schutzbereich der vorliegenden Erfindung.

## Patentansprüche

1. Biopsienadel zum Entnehmen einer Biopsieprobe aus dem Körper eines Patienten, mit einer eine Achse (A) aufweisenden Außenkanüle (44), einer rohrförmigen Innenkanüle (42), die zu der Außenkanüle (44) koaxial und dieser gegenüber verschiebbar ist und in der ein Stilett (10A bzw.10B) koaxial relativ zur Innenkanüle (42) verschiebbar angeordnet ist, wobei das Stilett eine Außenfläche (30), ein distales Ende (12), einen proximalen Teil (14) und eine zwischen dem Ende (12) und dem Teil (14) angeordnete Probenaufnahmekammer (32) zur Aufnahme einer Probe (S) an einer Stelle des Körpers aufweist, wobei sich die Aufnahmekammer (32) radial in das Stilett (10A) von dessen Außenfläche (30) aus öffnet und eine sich in Axialrichtung erstreckende Bodenwand (34) und im axialen Abstand voneinander angeordnete distale und proximale Stirnwände (38 bzw. 40) aufweist, die sich zwischen der Bodenwand (34) und der Außenfläche (30) des Stiletts (10A) erstrecken und für die Aufnahmekammer (32) eine Eintrittsöffnung bilden, die sich in Radialrichtung im Abstand von der Bodenwand (34) befindet, **dadurch gekennzeichnet, daß** sowohl die distale Stirnwand (38 bzw. 64) als auch die proximale Stirnwand (40 bzw. 66) von der Eintrittsöffnung der Aufnahmekammer (32) aus in Bezug auf das entsprechende distale Ende (12) bzw. den proximalen Teil (14) des Stilettes (10A bzw. 10B) zurückspringend ausgebildet ist, und daß das Stilett um seine Längsachse drehbar in der rohrförmigen Innenkanüle (42) aufgenommen ist.

2. Biopsienadel nach Anspruch 1, **dadurch gekennzeichnet, daß** mindestens eine Stirnwand (38 bzw. 40) in einem spitzen Winkel (a) zu der Bodenwand (34) geneigt ist.

3. Biopsienadel nach einem der Ansprüche 1 oder 2, **dadurch gekennzeichnet, daß** sowohl die distale Stirnwand (38) als auch die proximale Stirnwand (40) in einem spitzen Winkel zur Bodenwand (34) geneigt ist.

4. Biopsienadel nach einem der Ansprüche 1 bis 3, **dadurch gekennzeichnet, daß** der spitze Winkel (a) etwa 45° beträgt.

5. Biopsienadel nach einem der Ansprüche 1 bis 4, **dadurch gekennzeichnet, daß** mindestens eine Stirnwand (64 bzw. 66) zwischen der Eintrittsöffnung und der Bodenwand (34) gebogen ist.

6. Biopsienadel nach einem der Ansprüche 1 bis 5, **dadurch gekennzeichnet, daß** sowohl die distale Stirnwand (64) als auch die proximale Stirnwand (66) zwischen der Eintrittsöffnung und der Bodenwand (34) gebogen ist.

7. Biopsienadel nach einem der Ansprüche 1 bis 4, **dadurch gekennzeichnet, daß** sowohl die distale Stirnwand (48) als auch die proximale Stirnwand (40) eben und in einem spitzen Winkel zur Bodenwand (34) geneigt ist.

## Claims

1. A biopsy needle for taking a biopsy sample from the body of a patient, comprising an outer cannula (44) having an axis (A), a tubular inner cannula (42), which is coaxial with the outer cannula (44) and is displaceable in relation thereto, and in which a stylet (10A; 10B) is disposed coaxially in relation to the inner cannula (42), wherein the stylet comprises an outer face (30), a distal end (12), a proximal part (14) and a sampling chamber (32) disposed between the end (12) and the part (14) for taking a sample (S) at a location of the body, wherein the sampling chamber (32) opens out radially into the stylet (10A) from the outer face (30) thereof and comprises a basal wall (34) extending in an axial direction, and distal and proximal end walls (38; 40) which are disposed at an axial spacing from each other, which extend between the basal wall (34) and the outer face (30) of the stylet (10A), and which form an inlet opening for the sampling chamber (32), which inlet opening is situated at a radial spacing from the basal wall (34), **characterised in that** both the distal end wall (38; 64) and the proximal end wall (40; 66) are constructed standing back from the inlet opening of the sampling chamber (32) with respect to the corresponding distal end (12) or proximal part (14) of the stylet (10A; 10B), and that the stylet is accommodated in the tubular inner cannula (42) so that it can rotate about its longitudinal axis.

2. A biopsy needle according to claim 1, **characterised in that** at least one end wall (38; 40) is inclined at an acute angle (a) to the basal wall (34).

3. A biopsy needle according to either one of claims 1 or 2, **characterised in that** both the distal end wall (38) and the proximal end wall (40) are inclined at an acute angle to the basal wall (34).

4. A biopsy needle according to any one of claims 1 to 3, **characterised in that** the acute angle (a) is about 45°.

5. A biopsy needle according to any one of claims 1 to 4, **characterised in that** at least one end wall (64 or 66) is curved between the inlet opening and the basal wall (34).

6. A biopsy needle according to any one of claims 1 to 5, **characterised in that** both the distal end wall (64) and the proximal end wall (66) are curved between the inlet opening and the basal wall (34).

7. A biopsy needle according to any one of claims 1 to 4, **characterised in that** both the distal end wall (48) and the proximal end wall (40) are flat and are inclined at an acute angle to the basal wall (34).

## Revendications

1. Aiguille de biopsie pour le prélèvement d'un échantillon de biopsie du corps d'un patient, comportant une canule externe (44) présentant un axe (A), une canule interne (42) tubulaire, qui est coaxiale à la canule externe (44) et qui peut coulisser par rapport à celle-ci et dans laquelle un stylet (10A, respectivement 10B) est disposé de manière à pouvoir coulisser axialement et par rapport à la canule interne (42), le stylet présentant une surface externe, une extrémité distale (12), une partie proximale (14) et une chambre de réception d'échantillon (32) disposée entre l'extrémité (12) et la partie (14), destinée à recevoir un échantillon (S) en un endroit du corps, la chambre de réception (32) s'ouvrant radialement dans le stylet (10A), à partir de sa surface externe (30) et présentant une paroi de fond (34) qui s'étend dans la direction axiale, ainsi que des parois frontales distales et proximales (respectivement 38 et 40) disposées à distance axiale l'une de l'autre, qui s'étendent entre la paroi de fond (34) et la surface externe (30) du stylet (10A) et forment une ouverture d'entrée de la chambre de réception (32), laquelle ouverture se trouve à distance de la paroi de fond (34) dans la direction radiale, caractérisée en ce que la paroi frontale distale (respectivement 38 et 64) ainsi que la paroi frontale proximale (respectivement 40 et 66) sont réalisées en retrait à partir de l'ouverture d'entrée de la chambre de réception (32), par rapport à l'extrémité distale (12) correspondante ou à la partie proximale (14) du stylet (respectivement 10A et 10B), et en ce que le stylet est logé dans la canule interne (42) tubulaire de manière à pouvoir tourner autour de son axe longitudinal.

2. Aiguille de biopsie selon la revendication 1, caractérisée en ce qu'au moins une paroi frontale (respectivement 38 et 40) est inclinée sous un angle aigu (a) par rapport à la paroi de fond (34).

3. Aiguille de biopsie selon la revendication 1 ou 2, caractérisée en ce que la paroi frontale distale (38) ainsi que la paroi frontale proximale (40) sont inclinées sous un angle aigu par rapport à la paroi de fond (34).

4. Aiguille de biopsie selon l'une des revendications 1 à 3, caractérisée en ce que l'angle aigu (a) est environ égal à 45°.

5. Aiguille de biopsie selon l'une des revendications 1 à 4, caractérisée en ce qu'au moins une paroi frontale (respectivement 64 et 66) est arquée entre l'ouverture d'entrée et la paroi de fond (34).

6. Aiguille de biopsie selon l'une des revendications 1 à 5, caractérisée en ce que la paroi frontale distale (64) ainsi que la paroi frontale proximale (66) sont arquées entre l'ouverture d'entrée et la paroi de fond (34).

7. Aiguille de biopsie selon l'une des revendications 1 à 4, caractérisée en ce que la paroi frontale distale (48) ainsi que la paroi frontale proximale (40) sont planes et inclinées sous un angle aigu par rapport à la paroi de fond (34).
